Europäisches Patentamt

European Patent Office

Office européen des brevets

(11) Numéro de publication: **0 216 660**
**A1**

## (12) DEMANDE DE BREVET EUROPEEN

(21) Numéro de dépôt: 86401805.6

(22) Date de dépôt: 13.08.86

(51) Int. Cl.⁴: **A 61 M 16/00**
F 16 L 55/16

(30) Priorité: 13.09.85 FR 8513580

(43) Date de publication de la demande:
01.04.87 Bulletin 87/14

(84) Etats contractants désignés:
BE CH DE FR GB IT LI SE

(71) Demandeur: Borrelly, Jacques
1, rue de la Vigne des Sables
F-54180 Heillecourt (FR)

(72) Inventeur: Borrelly, Jacques
1, rue de la Vigne des Sables
F-54180 Heillecourt (FR)

(74) Mandataire: Flavenot, Bernard
Société ABRITT 17, rue du Docteur Charcot La Norville
F-91290 Arpajon (FR)

(54) Dispositif d'étanchéité pour une tubulure.

㉗ La présente invention concerne un dispositif d'étanchéité.
Le dispositif se caractérise essentiellement par le fait qu'il comporte une âme cylindrique (1) dont la section affecte la forme d'un "U" et qui est réalisée en un matériau présentant une certaine élasticité de déformation de sa forme dans un plan perpendiculaire à la direction (2) des génératrices définissant le cylindre, une membrane élastique (5) disposée autour de l'âme centrale et associée de façon étanche à la paroi latérale (15) de l'âme centrale, pour former une chambre déformable (8) fermée ayant une section sensiblement en forme de croissant, et des moyens (9, 10) d'admission de luide sous pression à l'intérieur de ladite chambre (8).
Application, en particulier, à l'étanchéité périphérique d'une trachée-artère lors d'une ventilation mécanique d'un malade.

fig.1

**Description**

DISPOSITIF D'ETANCHEITE POUR UNE TUBULURE

La présente invention concerne les dispositifs d'étanchéité dans les tubulures, comme par exemple les dispositifs qui permettent d'obtenir l'étanchéité ou l'obturation par application d'un tampon, cette application se faisant par l'intérieur de la tubulure.

Dans de nombreux domaines, il arrive que l'on ait besoin, pour différentes raisons, de réaliser une étanchéité, par exemple en obturant des ouvertures dans des tubulures, que ces ouvertures aient été pratiquées volontairement ou pas. Or, dans certains cas, il n'est pas toujours facile de pouvoir accéder par l'extérieur à une ouverture de la tubulure, afin d'appliquer sur cette ouverture un tampon, de quelque nature qu'il soit, pour la boucher.

C'est ainsi que des techniques ont été mises au point pour effectuer cette opération d'étanchéité ou d'obturation par l'intérieur de la tubulure. Le tampon est alors appliqué sur l'ouverture, en ayant été introduit à l'intérieur de la tubulure comportant cette ouverture . Pour obtenir une bonne obturation par application d'un tampon sur une ouverture, il est concevable que la qualité de l'obturation dépend d'un certain nombre de critères, par exemple la nature des matériaux en présence, matériau dans lequel est réalisée l'ouverture et matériau du tampon. Mais il en existe un autre qui est la valeur de la force d'application du tampon, notamment sur les lisières ou bords de l'ouverture.

Quand l'application du tampon se fait par l'extérieur de la tubulure, il est généralement facile de donner à cette force la valeur voulue en trouvant un point d'appui relativement facile d'accès. Par contre, pour une obturation par l'intérieur de la tubulure, la détermination du point d'appui est relativement plus difficile à obtenir, surtout quand il est nécessaire, par exemple, que cette tubulure reste parcourue par un fluide quelconque, gazeux ou liquide.

Pour résoudre ce problème, on a déjà réalisé des structures d'obturation particulières. Ces structures comportent une âme centrale constituée par un manchon cylindrique rigide formant une portion de conduite continue, le diamètre extérieur de cette portion de conduite étant inférieur au diamètre intérieur de la tubulure à étancher. Une membrane souple et élastique est disposée autour de ce manchon et solidarisée, de façon étanche, à ses deux extrémités, aux bords extrèmes du manchon, de façon à former une chambre annulaire étanche déformable autour du manchon. Un petit tuyau d'admission de fluide, comme par exemple de gaz, sous pression est relié à l'intérieur de cette chambre annulaire, pour pouvoir la gonfler à volonté.

Pour pouvoir étancher une ouverture dans une tubulure, on introduit cette structure à l'intérieur de la tubulure en laissant accessible l'extrémité libre du petit tuyau à l'extérieur. Quand la membrane est juste placée en regard de l'ouverture, par l'intermédiaire du petit tuyau, on introduit un fluide sous une pression prédéterminée. De cette façon, la chambre annulaire se gonfle et, en se gonflant, par sa surface extérieure vient alors épouser toute la surface périphérique intérieure de la tubulure et celle se trouvant autour de l'ouverture et à son voisinage.

L'ouverture se trouve ainsi obturée par application d'un tampon constitué par la partie de membrane s'appuyant sur ses bords, la force d'application étant donnée par la pression du fluide dans la chambre annulaire et la surface d'appui engendrant la force de réaction, cette surface d'appui étant, en fait, constituée par toute la surface périphérique sur laquelle vient au contact la membrane formant la paroi extérieure de la chambre annulaire. Le manchon cylindrique rigide se trouve de ce fait sensiblement au centre de la tubulure et permet une continuité de celle-ci pour la circulation du fluide.

Cette technique donne de très bons résultats pour toutes les tubulures qui ont une paroi qui peut supporter la force de pression sur toute sa surface.

En revanche, il existe des tubulures qui ne peuvent pas supporter une telle force sur toute leur périphérie. Tel est le cas, par exemple, dans le domaine médical, de la trachée-artère.

En effet, on sait que la trachée-artère a une configuration cylindrique, mais absolument pas de révolution, et qu'elle comporte essentiellement deux parties. La première partie est en forme de goulotte, à section sensiblement en forme de "U", relativement rigide du fait même qu'elle comporte une armature. La seconde partie relie les deux bords longitudinaux de la goulotte. Cette seconde partie est fibreuse, très souple et même fragile.

Ainsi, dans le cas, par exemple, d'une ventilation forcée d'un malade par sa trachée-artère, une étanchéité périphérique avec une structure comme celle qui a été décrite est d'une utilisation très dangereuse à cause de la fragilité de la seconde partie de la trachée-artère.

Aussi, la présente invention a-t-elle pour but de réaliser un dispositif d'étanchéité pour tubulure, permettant d'éliminer l'inconvénient mentionné ci-dessus et qui trouve une application avantageuse, notamment, pour l'obturation d'une ouverture produite dans une tubulure et la réalisation d'une étanchéité périphérique dans le cas de la trachée-artère.

Plus précisément, la présente invention a pour objet un dispositif d'étanchéité pour tubulure, caractérisé par le fait qu'il comporte une âme centrale cylindrique dont la section affecte sensiblement la forme d'un "U", ladite âme centrale étant réalisée pour présenter une certaine élasticité de déformation de sa forme dans un plan perpendiculaire à la direction des génératrices définissant le cylindre, une membrane disposée autour de ladite âme centrale, ladite membrane étant réalisée en un matériau souple et élastique, son coefficient d'élasticité étant très supérieur à celui de ladite âme centrale, ladite membrane étant associée de façon étanche à la paroi latérale de ladite âme centrale, pour former une chambre déformable fermée ayant une section sensiblement en forme de croissant, et des moyens d'admission de fluide sous pression à l'intérieur de ladite chambre.

D'autres caractéristiques et avantages de la présente invention apparaîtront au cours de la description suivante donnée en regard des dessins annexés à titre illustratif mais nullement limitatif, dans lesquels :

- les Figures 1 et 2 représentent, dans deux coupes perpendiculaires, un mode de réalisation d'un dispositif selon l'invention,

- les Figures 3 et 4 illustrent une application du dispositif à l'obturation, par exemple, d'une ouverture créée dans une tubulure cylindrique,

- la Figure 5 illustre une application du dispositif à l'étanchéité périphérique dans une trachée-artère,

- la Figure 6 représente un autre mode de réalisation d'un dispositif selon l'invention permettant d'assurer, en plus, une fonction de canule de trachéotomie, et

- la Figure 7 représente, sous forme schématique et en coupe transversale, un mode de réalisation d'un élément essentiel du dispositif selon l'invention.

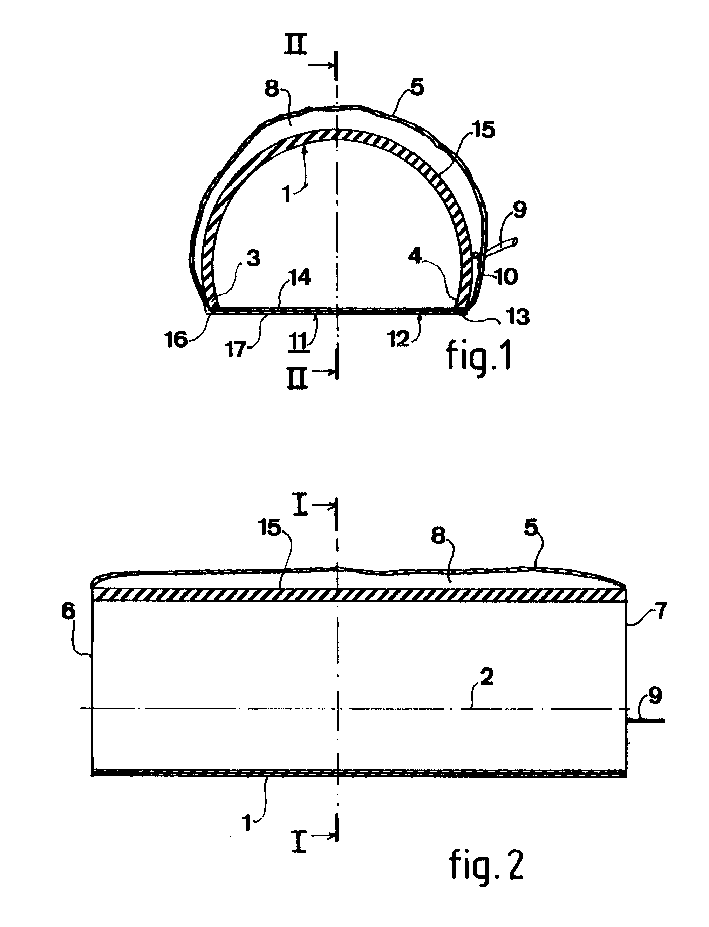

Les Figures 1 et 2 représentent deux coupes perpendiculaires d'un mode de réalisation d'un dispositif d'étanchéité selon l'invention, la Figure 1 représentant la vue en coupe référencée I-I sur la Figure 2, et la Figure 2 représentant la vue en coupe référencée II-II sur la Figure 1.

Le dispositif selon l'invention comprend une âme centrale 1 qui est de forme avantageusement cylindrique non fermée, dont la section affecte la forme d'une portion de cercle. Cette âme centrale est réalisée dans un matériau présentant une certaine élasticité de déformation dans un plan perpendiculaire à la direction des génératrices illustrée en 2 sur la Figure 2, le plan étant, en fait, parallèle au plan de coupe I-I, ou à celui de la Figure 1. Ce matériau peut être une matière semi-rigide préformée en portion de cylindre non fermée. De ce fait, lorsqu'une traction est exercée sur les deux bords longitudinaux 3 et 4 pour tendre à les éloigner, cette âme se déforme en s'ouvrant et, lorsqu'on relâche la traction, l'âme reprend sa forme initiale, comme représentée sur la Figure 1.

Sur cette âme centrale 1 est fixée une membrane souple et élastique 5. Cette membrane 5 est fixée sur le bord de l'âme centrale, aussi bien sur les deux bords longitudinaux 3 et 4 que sur les deux bords courbés 6 et 7 aux deux extrémités de l'âme centrale 1, de façon à former une chambre déformable 8 fermée ayant une section de forme générale sensiblement en croissant. Pour fermer cette chambre de façon qu'elle soit étanche, les bords de la membrane sont soit collés soit soudés aux bords de l'âme centrale.

Dans un mode avantageux pour certaines applications, les deux bords longitudinaux 3 et 4 de l'âme centrale 1 sont reliés entre eux par des moyens élastiques 11, pour agir comme tendeur.

Les moyens élastiques peuvent être constitués par un ressort de traction relié aux deux bords 3 et 4 qui tend à s'opposer à l'écartement des deux bords quand, par une action quelconque, ils ont tendance à s'éloigner.

Ces moyens élastiques 11 peuvent aussi être constitués par une membrane de liaison 12 qui peut être la même membrane que la membrane 5 formant avec l'âme 1 la chambre 8. Dans ce cas, la membrane 5 est constituée par un manchon cylindrique fermé entourant complètement l'âme centrale. Cependant, cette membrane sera fixée de façon étanche aux deux bords longitudinaux 3 et 4 de l'âme centrale pour former, par une partie, la chambre étanche 8 et, par la partie restante relativement tendue, les moyens élastiques 11.

Dans un autre mode avantageux, cette membrane 12 peut être constituée par la superposition de deux portions de membrane qui, en fait, n'est que le recouvrement des deux extrémités d'une seule.

En fait, pour réaliser ce dispositif, on prend une membrane ayant une longueur supérieure à la valeur de la longueur de la périphérie de la section de l'âme centrale 1. On fixe une extrémité 13 de cette membrane sur le bord 4, on tend linéairement la membrane entre les deux bords 4 et 3, on fixe cette portion de membrane 14 sur la bord 3, puis on entoure la surface extérieure courbe 15 de l'âme centrale 1 pour revenir positionner la membrane sur le bord 4 et recouvrir la première portion de membrane 14 tendue, on fixe la deuxième extrémité 16 de la membrane sur la portion de membrane 14 au niveau où celle-ci est elle-même fixée sur le bord longitudinal 3.

De cette façon, la membrane de liaison 12 située entre les deux bords 3 et 4 a un coefficient d'élasticité supérieur à celui d'une seule membrane unitaire et présente, en plus, une solidité qui peut être intéressante dans certaines applications.

Un petit tuyau 9 a une extrémité 10 connectée à l'intérieur de la chambre 8, de façon étanche et de façon à pouvoir introduire, comme il sera explicité ci-après, un fluide avec une pression déterminée et gonfler cette chambre 8.

Ci-dessus, il vient d'être décrit un mode de réalisation d'un dispositif d'étanchéité par l'intérieur d'une tubulure comportant une ouverture comme, par exemple, une fente.

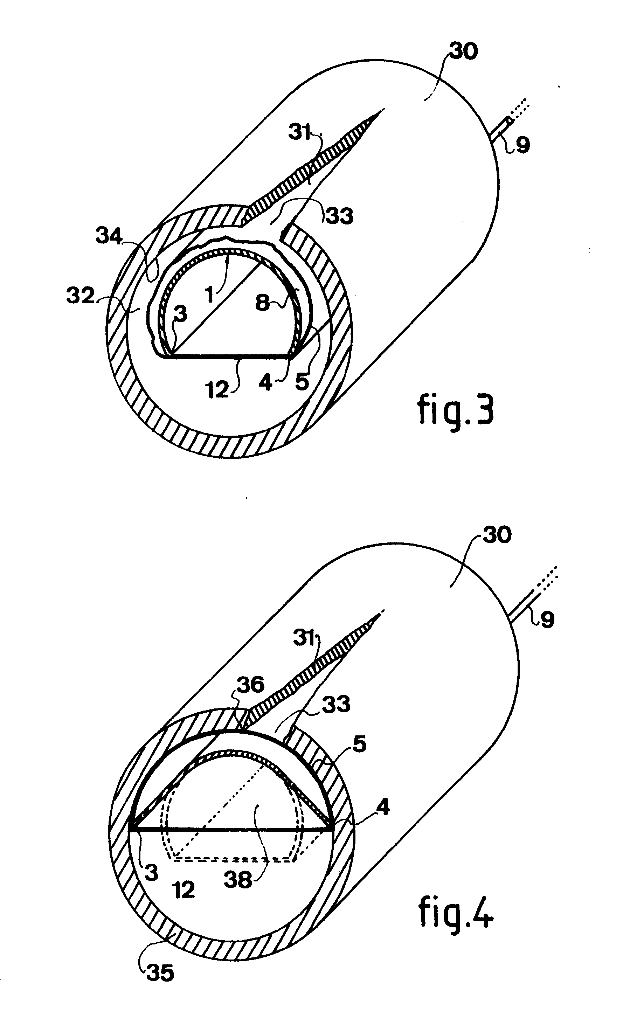

Les Figures 3 et 4 représentent le dispositif dans une application d'étanchéité d'un tube 30 comportant une fente 31, ce tube ayant une partie 35 de sa paroi sur laquelle il est déconseillé de prendre appui.

Ces deux Figures 3 et 4 représentent le dispositif d'étanchéité en coupe transversale, dans deux positions différentes, la première (Figure 3) quand le dispositif est introduit dans le tube, la seconde (Figure 4) quand le dispositif obture la fente 31.

Sur les Figures 3 et 4 sont représentées deux étapes dans le processus d'obturation de la fente 31 dans le tube 30. La dimension du dispositif est suffisante pour que la membrane 5 soit d'une dimension nettement supérieure à la section de la fente 31. Le dispositif introduit est maintenu de telle façon que la membrane 5 soit à proximité de cette fente 31, du moins de son ouverture 33 située sur la paroi intérieure 34 du tube 30. La partie plane 12 située entre les deux bords 3 et 4 est, elle, en regard de la partie du tube qui présente des points de faiblesse et sur laquelle il ne doit pas être pris appui.

L'extrémité du petit tuyau 9 est laissé libre à l'extérieur. Par différents moyens, cette membrane 5 avec l'âme centrale 1 sont maintenues à proximité de cette fente 31. Quand le dispositif est positionné comme représenté sur la Figure 3, par le tuyau 9 on insuffle, par exemple, du gaz sous pression qui tend, de ce fait, à gonfler la chambre déformable 8. Dans un premier temps, la membrane 5 se tend, puis, sous l'action de la pression augmentant à l'intérieur de la chambre 8, l'âme centrale 1 se déforme de telle façon que ses deux bords 3 et 4 tendent à s'éloigner en tirant sur la membrane élastique 12 les reliant. En augmentant progressivement cette pression, ces deux bords 3 et 4 viennent au contact de la paroi périphérique intérieure 34 du tube 30, la membrane 12 se tend en restant éloignée de la partie 35 du tube qui est relativement fragile. Ensuite, une fois que ces deux bords 3 et 4 sont venus au contact de la paroi périphérique cylindrique 34 intérieure du tube 30, la membrane peut subir encore une déformation du fait de l'insufflation de ce gaz sous pression, de telle façon qu'elle épouse parfaitement toute la surface intérieure 34 du tube 30 et, donc, tout le bord latéral 36 entourant l'ouverture 33 de la fente 31 se trouvant sur la surface 34.

La pression sera ajustée de telle façon que le dispositif reste bien plaqué contre la paroi 34 du fait, notamment, des forces de réaction et de frottement, mais qu'en aucune façon il vienne se déformer pour aller appuyer, par exemple, sur la partie plus fragile 35 du tube 30. La déformation qui tend à ouvrir l'âme centrale 1 lorsqu'on gonfle la chambre 8 est un phénomène physique connu et, par l'effet de gonflement de la chambre, la membrane s'applique parfaitement contre la paroi intérieure 34 du tube pour venir au contact des bords de la fente qu'elle obture alors parfaitement.

On voit donc que, dans ces conditions, la membrane 5 constitue un tampon qui obture parfaitement la fente 31, tandis que la membrane 12 ne prend en aucune façon appui sur la partie 35 du tube 30. Cette partie 35 est, par exemple, une partie fragile qui ne présente pas une rigidité suffisante pour pouvoir constituer une surface d'appui pouvant engendrer une force de réaction pour maintenir le tampon contre la fente 31. Seule la surface 34 qui est une portion de paroi intérieure du tube 30 produit cette force de réaction qui maintient la membrane 5 contre elle et sur la fente 31. Le dispositif permet ainsi d'obturer parfaitement la fente 31 située dans le tube 30, par l'intérieur de celui-ci.

Le mode d'utilisation et d'application à l'étanchéité d'un tube comportant une fente, qui vient d'être décrit, d'un dispositif selon l'invention n'est donné qu'à titre indicatif et illustratif, dans un domaine d'application générale. En revanche, ce dispositif trouve une application avantageuse pour réaliser une étanchéité périphérique à une trachée-artère, dans le but de pouvoir ventiler artificiellement un malade.

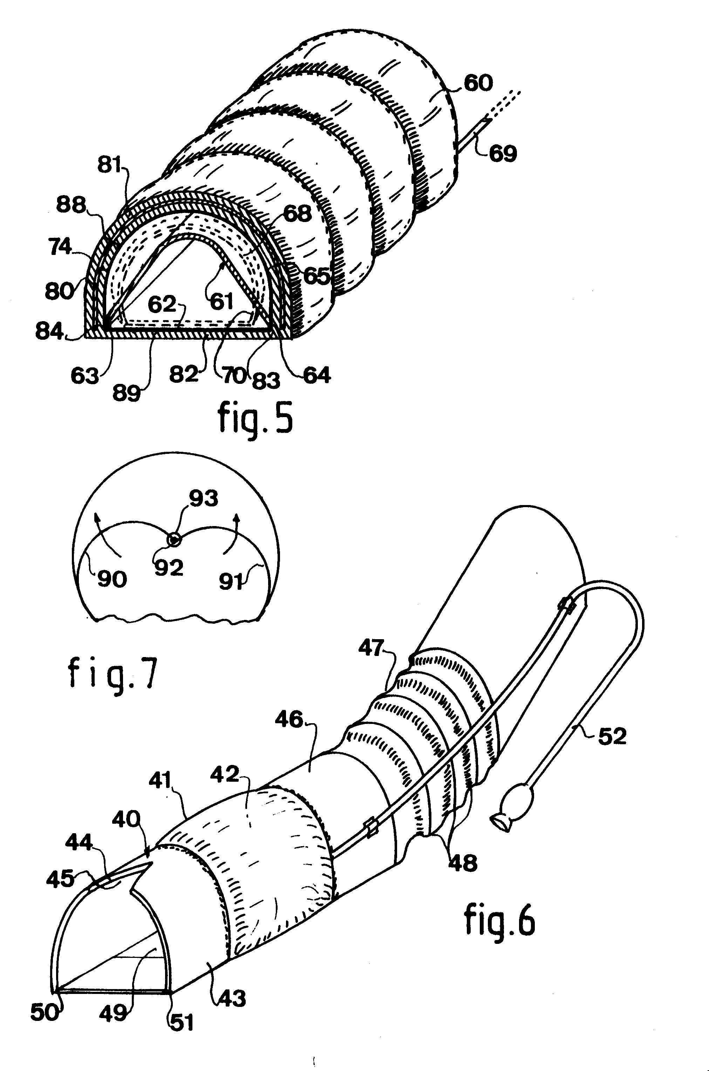

La Figure 5 représente un mode de réalisation du dispositif d'étanchéité dans une application à l'étanchéité périphérique dans une trachée-artère. Dans ce cas, le dispositif est introduit dans la trachée-artère 60, dans la forme telle que représentée en pointillé 70. Quand le dispositif a pris un telle position, par l'extrémité du conduit 69 restée à l'extérieur, on introduit un fluide en pression contrôlée, de façon que la chambre en croissant 68 se gonfle et que la membrane 65 se plaque contre la paroi intérieure 74 de la trachée-artère 60, pendant que l'âme centrale 61 se déforme comme expliqué ci-avant. Cette déformation de l'âme centrale 61 se fait de manière que ses deux bords longitudinaux 63 et 64 viennent se positionner le plus près possible des coins angulaires 83 et 84 intérieurs à la trachée-artère formés par l'intersection de la paroi semi-circulaire 81 comportant son anneau semi-circulaire 80 qui constitue un renfort de cartilage, avec la paroi sensiblement plane fibreuse 82. Ainsi, la membrane 62 reliant les deux bords 63 et 64, de façon sensiblement rectiligne, est parallèle et au contact de cette paroi fibreuse 82, en l'effleurant juste, de manière à réaliser une étanchéité latérale par friction pour éviter de déformer cette paroi fibreuse qui, comme chacune le sait, est très fragile.

De ce fait, on peut alors réaliser une ventilation du type mécanique d'assistance respiratoire d'un malade, en insufflant l'air entre la membrane 62 et l'âme centrale 61, sans qu'il se produise des fuites entre la paroi latérale longitudinale entourant le dispositif et la surface intérieure de la trachée-artère, que ce soit au niveau de la surface de contact 88 entre la surface extérieure de la membrane en croissant 65 et la surface intérieure 74 de la paroi semi-circulaire 81, ou au niveau de la surface de contact 89 entre la surface extérieure de la membrane 62 reliant de façon sensiblement rectiligne les deux bords 63 et 64 de l'âme centrale 61 et la surface intérieure de la paroi fibreuse 82.

On voit donc l'avantage d'un tel dispositif dans le cas de l'application à une trachée-artère, notamment pour une assistance respiratoire, ce dispositif permettant de pouvoir insuffler l'air entre l'âme centrale 61 et la membrane 62, sans que se produisent des fuites, et sans déformation de la paroi 82 fibreuse et fragile.

La Figure 6 représente un autre mode de réalisation d'un dispositif d'étanchéité comme représente sur les Figures 1 à 4. Mais, dans ce cas, la longueur de l'âme centrale 40 est beaucoup plus grande que celle de membrane 41. Cette âme 40 dépasse ainsi de chaque côté de la chambre déformable 42.

La partie 43 qui est à une extrémité de la chambre 42 dépasse celle-ci d'une relativement faible quantité permettant de constituer une butée 44 qui peut venir se positionner contre un élément qui se trouve à l'intérieur de la tubulure à étancher. Dans le cas de la trachée-artère, cette extrémité peut éventuellement comporter une encoche 45 pour venir se positionner plus facilement à la bifurcation de deux bronches souches. Dans ce cas, cette butée est avantageusement en un matériau souple pour constituer une butée sans danger, en même temps qu'un moyen d'étanchéité.

En revanche, la partie 46 qui est à l'autre extrémité de la chambre peut être d'une grande longueur, et même comporter des articulations 47 avec certains degrés de rotation suivant les cas. Ces articulations 47 peuvent être constituées par une pluralité d'ondulations 48 réalisées dans la paroi de l'âme centrale pour constituer un soufflet. Cette configuration permet de pouvoir introduire dans la tubulure à étancher le dispositif d'obturation, même si celle-ci comporte des endroits ayant une certaine courbure. Cette réalisation trouve encore une application avantageuse dans le domaine de la ventilation artificielle d'un malade par trachéotomie. Dans ce cas, le soufflet est avantageusement situé à la jonction entre la portion du dispositif introduite dans la trachée-artère et la portion extérieure de ce dispositif.

Comme mentionné précédemment, le dispositif d'étanchéité comporte avantageusement une membrane tendeur 49 entre les deux bords 50 et 51 de l'âme centrale 40 en forme de gouttière, pour compenser la force due à la déformation quand la chambre déformable 42 est mise sous pression. Cette membrane 49 peut être allongée, dans certaines conditions, pour fermer complètement la gouttière et en faire un conduit tubulaire sur toute sa longueur. Ce conduit peut alors, dans le même objet du dispositif selon l'invention, constituer un moyen pour introduire dans cette tubulure un autre fluide, pour quelque raison que ce soit, et constituer de ce fait, par exemple un canule de trachéotomie permettant, par exemple, de ventiler le malade. De même, à la chambre déformable est aussi associé un tuyau d'insufflation 52 qui permet, comme cela a été expliqué précédemment en regard des Figures 1 et 2, de gonfler la chambre en "U" 42, ce tuyau courant par exemple le long de la partie prolongée 46.

Dans un mode particulier de réalisation, quand on connaît avec une assez bonne précision les dimensions intérieures du tube à étancher, la membrane 49 peut être constituée par des moyens de liaison non élastiques ayant une longueur maximale donnée, mais pouvant par exemple se plier pour éviter une déformation du tube au niveau des deux extrémités 3 et 4 de l'âme centrale 1. La longueur maximale de la membrane est alors égale à celle de la corde reliant l'intérieur du tube 30 entre les deux points 3 et 4 (Fig. 4) ou 83 et 84 (Fig. 5).

Enfin, dans un autre mode de réalisation, l'âme centrale peut être avantageusement constituée, comme illustré schématiquement sur la Figure 7, au moins de deux parties cylindriques de révolution 90 et 91 accouplées de façon étanche par des moyens d'articulation 93, du type charnière, suivant une de leurs génératrices 92. Cette articulation permet à l'âme centrale de se déformer d'une façon équivalente à une déformation élastique.

## Revendications

1. Dispositif d'étanchéité pour tubulure (30, 60), CARACTERISE PAR LE FAIT QU'il comporte :
- une âme centrale cylindrique (1, 40, 61) dont la section affecte sensiblement la forme d'un "U", ladite âme centrale étant réalisée pour présenter une certaine élasticité de déformation de sa forme dans un plan perpendiculaire à la direction (2) des génératrices définissant le cylindre,
- une membrane (5, 41, 65) disposée autour de ladite âme centrale, ladite membrane étant réalisée en un matériau souple et élastique, son coefficient d'élasticité étant très supérieur à celui de ladite âme centrale, ladite membrane étant associée de façon étanche à la paroi latérale (15) de ladite âme centrale, pour former une chambre déformable (8, 42, 68) fermée ayant une section sensiblement en forme de croissant, et
- des moyens (9, 10, 69, 52) d'admission de fluide sous pression à l'intérieur de ladite chambre (8, 42, 68).

2. Dispositif selon la revendication 1, CARACTERISE PAR LE FAIT QU'il comporte des moyens de ressort élastiques en traction (11) reliant les deux bords longitudinaux (3-4, 50-51, 63-64) de ladite âme centrale (1, 40, 61) en "U".

3. Dispositif selon la revendication 2, CARACTERISE PAR LE FAIT QUE lesdits moyens de ressort (11) sont constitués par une paroi (12, 62, 49) élastique.

4. Dispositif sleon la revendication 3, CARACTERISE PAR LE FAIT QUE ladite paroi élastique (12) est constituée d'une partie (14, 17) de ladite membrane (5, 41) entourant ladite âme centrale.

5. Dispositif selon la revendication 3, CARACTERISE PAR LE FAIT QUE ladite paroi élastique (12) est constituée par le recouvrement de deux parties d'extrémités (14, 17) de ladite membrane (5).

6. Dispositif selon la revendication 1, CARACTERISE PAR LE FAIT QUE la longueur de ladite âme centrale (40) est supérieure à la longueur de ladite chambre (42).

7. Dispositif selon la revendication 6, caractérisé par le fait qu'une partie (43) de ladite âme centrale (40) située d'un côté de ladite chambre (42) comporte des moyens de butée (44).

8. Dispositif selon la revendication 6, caractérisé par le fait qu'une partie (46) de ladite âme centrale (40) située d'un côté de ladite chambre (42) comporte des moyens d'articulation (47).

9. Dispositif selon la revendication 8, caractérisé par le fait que lesdits moyens d'articulation (47) sont constitués par une partie de la paroi de ladite âme centrale (40) comportant des ondulations (48) formant soufflet.

10. Dispositif selon l'une des revendications précédentes, caractérisé par le fait que les deux bords longitudinaux (3-4, 50-51, 63-64) sont reliés par une membrane d'étanchéité sensiblement sur toute leur longueur.

11. Dispositif selon l'une des revendications précédentes 3 à 10, caractérisé par le fait que, dans le cas d'une étanchéité périphérique d'une trachée-artère, la longueur de ladite âme centrale (61) est déterminée pour que les deux bords (63, 64) longitudinaux de cette dite âme viennent au contact respectivement des deux coins angulaires (84,83) formés par l'intersection de la paroi semi-circulaire (81) et de la paroi plane fibreuse (82) de façon que la membrane (62) reliant les deux bords longitudinaux soit apte à être tangente à la surface intérieure de ladite paroi plane fibreuse (82).

12. Dispositif selon la revendication 1, caractérisé par le fait qu'il comporte des moyens de liaison reliant les deux bords longitudinaux (3-4, 50-51, 63-64) de ladite âme centrale (1, 40, 61) en "U", lesdits moyens de liaison déterminant une longueur maximale.

13. Dispositif selon l'une des revendications précédentes, CARACTERISE PAR LE FAIT QUE ladite âme centrale est constituée au moins de deux parties cylindriques (90, 91) sensiblement de révolution, et de moyens d'articulation étanches (93) des deux dites parties cylindriques.

fig.1

fig.2

fig.3

fig.4

fig.5

fig.7

fig.6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| A | FR-A-2 204 431 (MEDICAL PRODUCTS CORP.) * Revendication 1; figures 1-5 * | 1 | A 61 M 16/00 F 16 L 55/16 |
| A | FR-A-1 576 397 (MILLET PUIG) * Figure 1 * | 1 | |
| A | US-A-1 928 542 (M. REITERER) | | |
| A | US-A-3 298 399 (E.A. SLADE) | | |
| | ----- | | |

|  |
|---|
| **DOMAINES TECHNIQUES RECHERCHES (Int Cl.4)** |
| A 61 M F 16 L |

Le présent rapport de recherche a été établi pour toutes les revendications

| Lieu de la recherche | Date d'achèvement de la recherche | Examinateur |
|---|---|---|
| LA HAYE | 26-09-1986 | ANGIUS P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant